# EUROPEAN PATENT SPECIFICATION

(11) **EP 4 052 640 B1**
(45) Date of publication and mention of the grant of the patent: **07.06.2023**
(21) Application number: 21020130.7
(22) Date of filing: 06.03.2021
(51) Int. Cl.: A61B 5/01, A61B 5/00, A61B 5/11, G01J 5/00

(54) **HANDHELD DEVICE FOR MEASURING BODY TEMPERATURE**
TRAGBARE VORRICHTUNG ZUR MESSUNG DER KÖRPERTEMPERATUR
DISPOSITIF PORTATIF POUR MESURER LA TEMPÉRATURE CORPORELLE

(43) Date of publication of application: 07.09.2022
(73) Proprietor: Sidly Sp. z o.o., 00-020 Warszawa (PL)
(72) Inventor: Pizon, Michal, 26-411 Rusinów (PL); Kocyk, Edyta, 26-902 Grabów nad Pilica (PL)
(74) Representative: Adamczyk, Piotr

(56) References cited:
- CN-A- 111 685 457
- US-A1- 2016 113 517
- US-A1- 2020 394 387

## Description

The object of the invention is a wrist-worn device for measuring body temperature, worn by a patient on the wrist.

Various devices measuring vital signs have been known and used for many years. Publication No. KR20160006409 discloses a silicone wristband that displays the current body temperature and heart rate of a person wearing the wristband. In this solution, temperature measurement is performed by an indeterminate sensor located in the wristband, remaining in contact with the skin of the wrist.

Publication No. CN104706329 discloses a wristband for remote temperature measurement, in particular of young children. The wristband contains a thermistor sensor that is in contact with the skin of the wrist and a Bluetooth two-way communication module. The wristband's measuring system, after receiving a wireless call from a central unit, e.g. at a hospital, measures the temperature of the wrist and transmits it to the aforementioned central unit.

However, it is known that measuring the temperature of the entire body on the basis of the temperature of the wrist alone, although the simplest, is inaccurate. A consequence of this fact is, for example, the solution disclosed in publication No. CN106923796. It describes a wristband with several thermistor sensors and a wireless communication module. Using such a wristband, a large number of wrist temperature measurements are first taken on a number of patients while simultaneously recording the temperature measured under the armpit with traditional medical thermometers on the same patients. Then, using statistical methods, a mathematical model is built, which interprets (corrects) the result of wrist thermistor measurement so that it reflects the actual body temperature as accurately as possible.

US2016/113517A1 discloses a wrist-worn device for measuring body temperature. The device comprises a housing with means for securing it to the wrist of a patient. Inside the housing at least there is a microcontroller, a passive infrared sensor, a wireless communication module and accelerometer. The housing contains a measurement window for the passive infrared sensor. located on the housing's surface that is not intended for contact with the wrist.

The purpose of the invention was to improve the accuracy of body temperature measurements made with a wrist-worn device.

This purpose is fulfilled by the device according to the invention, comprising a housing with means for securing it to the wrist of a patient. Inside the housing of this device there is at least a microcontroller and, signal-connected to it, a temperature transducer and a wireless communication module. The invention comprises a passive infrared sensor as a temperature transducer. The housing contains a measurement window for the passive infrared sensor. The measurement window is located on the housing's surface that is not intended for contact with the wrist. The housing also contains an accelerometer signal-connected to a microcontroller. The accelerometer is configured to continuously record the trajectory of the device and the microcontroller is configured to read the temperature only if this trajectory corresponds to the lifting of the device near the head of the patient.

In one of variants of the invention, the measurement window of the housing faces the direction opposite to the wrist.

In next variant of the invention, the resolution of the accelerometer falls in the range from 0.0005 g to 0.05 g.

In next variant of the invention, the resolution of the passive infrared sensor falls in the range from 0.01°C to 0.52°C.

In next variant of the invention, the wireless communication module contains a GSM/GPRS communication block and a WiFi communication block operating in the band from 2,400 to 2,485 GHz.

In next variant of the invention, visual and acoustic signalling means are contained in the device housing.

In next variant of the invention, microcontroller control means are contained in the device housing.

In another variant of the invention, the visual signalling means are RGB luminescent LEDs.

In yet another variant of the invention, the acoustic signalling means is a loudspeaker.

The invention combines the advantages of a traditional thermometer wristband and an accurate medical thermometer, as it maintains the simplicity of the measurement itself while ensuring accuracy and reliability not achievable with other wrist-worn solutions. Thanks to a passive infrared sensor incorporated in the device according to the invention, the user can measure their body temperature at any time. The location of the measurement window in the front part of the housing of this device and taking the measurement at the moment of lifting the hand to the forehead make it possible to measure the forehead temperature, which is representative for the entire body. The measurement algorithm implemented by the microcontroller can detect a significant difference between the temperature of the object at which the infrared sensor is aimed at a given moment and the ambient temperature. This avoids accidental or misleading measurements in case of an unintentional hand raising gesture.

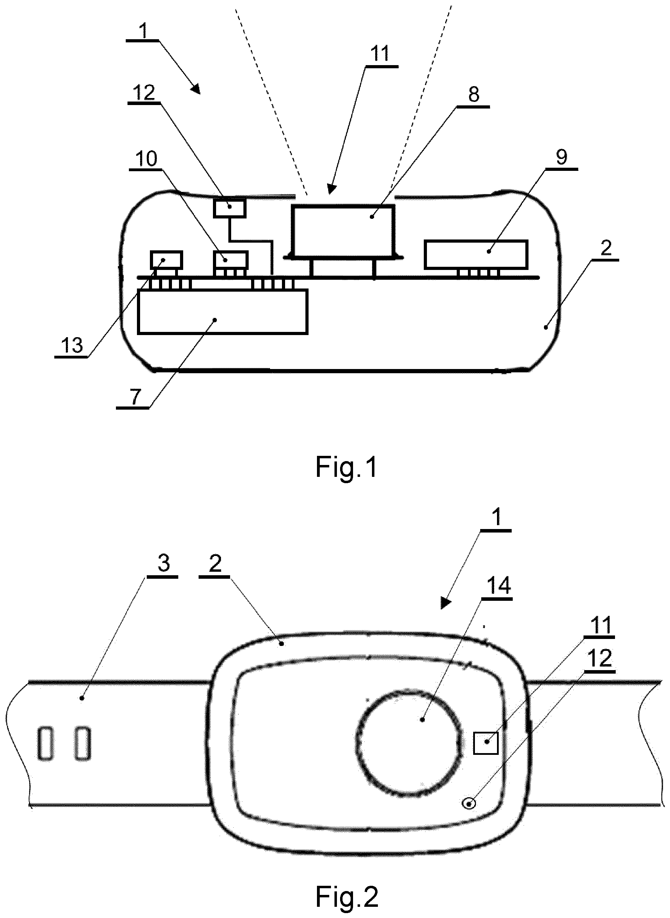

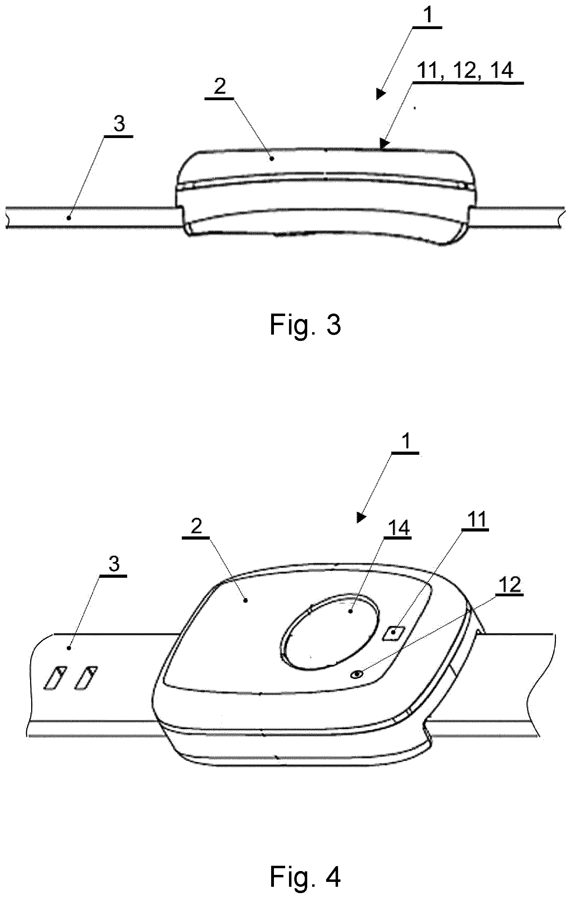

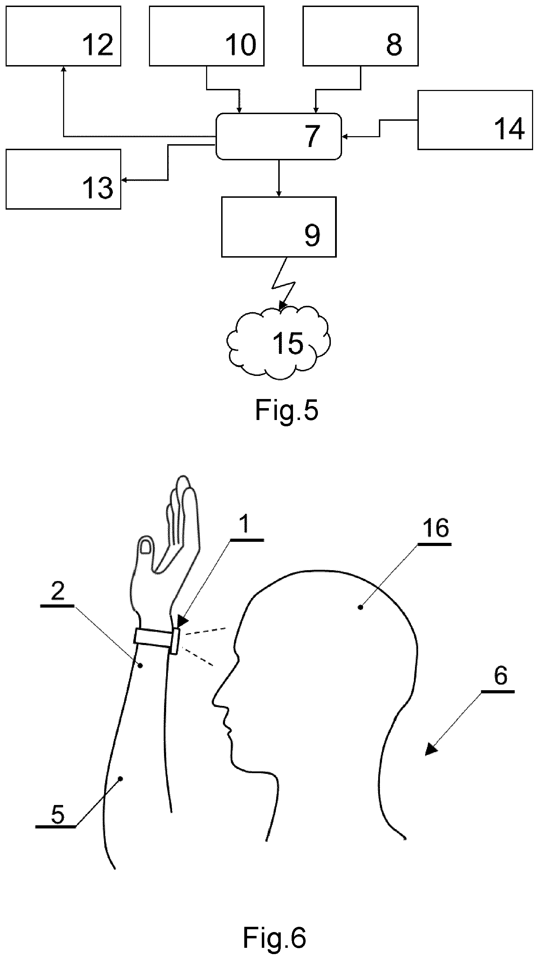

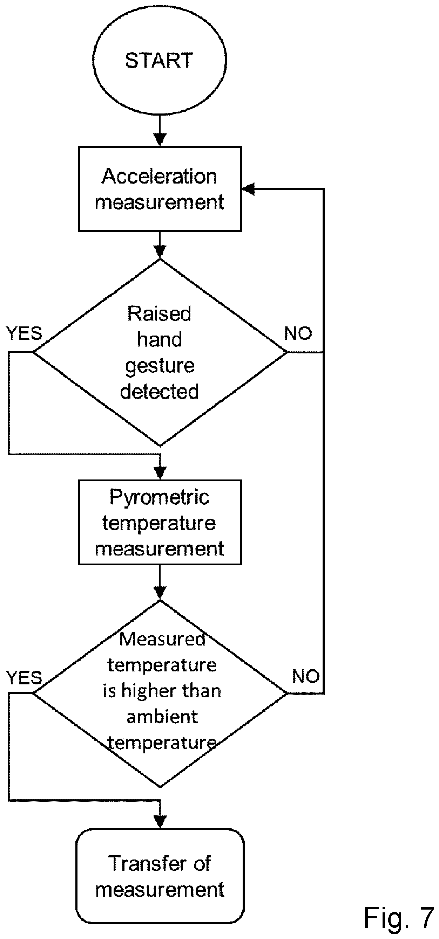

An exemplary embodiment of the invention is described in detail below and shown in the accompanying drawings. Fig.1 schematically shows a crosssection through the housing of the device according to the invention in the place where the measurement window of the temperature sensor is located, while Fig.2, Fig.3, and Fig.4 show the device according to the invention in orthogonal projection from above, in orthogonal projection from the side, and in axonometric projection, respectively. Fig.5 shows a block diagram of the measuring system of the device according to the invention. Fig.6 is a schematic representation of a patient during temperature measurement, while Fig.7 shows the measurement algorithm implemented by the device according to the invention.

The exemplary embodiment of the device according to the invention 1 has a housing 2 made of plastic and resembling the case of a known wristwatch. The housing 2 is provided with a known strap 3 for securing the device 1 on the wrist 4 of the hand 5 of a patient 6. The housing 2 includes a microcontroller 7, a temperature transducer 8, a wireless communication module 9, and an accelerometer 10, signal-connected to each other. In the described embodiment, an Arduino Pro Mini 328 IC is used as the microcontroller 7, while the transducer 8 is a Melexis MLX90313 passive infrared sensor with a resolution of 0.2°C and a range of 30 cm. On the upper side, that is, opposite to that which is in contact with the wrist 4 of the patient, the housing 2 contains a measurement window 11 in which the sensor 8 is located. A tri-axis Kionix KX003-1077 IC is used as the accelerometer 10, with a resolution of 0.00097 g in the direction of each axis. The mentioned resolutions of the transducer 8 and the accelerometer 10 are only examples, preferably being in the range of 0.01°C to 0.5°C and 0.0005 g to 0.05 g, respectively. The communication module 9 contains a GSM/GPRS block and a WiFi module. The GSM/GPRS module allows the device 1 to communicate via the mobile phone network operating at frequencies 850/900 and 1,800/1,900 MHz, while the WiFi module operates in the band from 2,400 to 2,485 GHz. The housing 2 also contains a visual signalling means 12, for example in the form of an RGB electroluminescent LED, and an acoustic signalling means 13, for example in the form of a miniature speaker. A button 14 is also provided on the housing 2 to manually control the microcontroller 7. The microcontroller 7 determines the current ambient temperature by averaging the readings taken by the sensor 8 at fixed intervals. Each movement of the hand 5 with the device 1 is recorded by the accelerometer 10. When such movement is detected, the microcontroller 7 reads the temperature as measured by the temperature sensor 8. If the measured temperature is different from the designated ambient temperature, such reading is transmitted to the communication module 9 and from there outside, for example to a data cloud 15 or to a hospital receiver (not shown in the drawing). It is also possible to continuously record the trajectory of the device 1 and to read the temperature only if this trajectory corresponds with a high probability to the lifting of the device 1 near the head 16 of the patient 6. The signalling means 12 and 13 are used to signal the start and end of the temperature measurement, while the button 14 allows the patient to call for help.

## Claims

1. A wrist-worn device (1) for measuring body temperature, comprising a housing (2) with means for securing (3) it to the wrist (4) of a patient, and having inside the housing (2) at least a microcontroller (7) and, signal-connected to it, a temperature transducer (8) and a wireless communication module (9), wherein a passive infrared sensor is the temperature transducer (8), the housing (2) contains a measurement window (11) for the passive infrared sensor (8), the window (11) is located on the housing's (2) surface that is not intended for contact with the wrist (4) and the housing (2) contains an accelerometer (10) signal-connected to the microcontroller (7), and wherein the accelerometer (10) is configured to continuously record the trajectory of the device (1, **characterized in that** the microcontroller (7) is configured to read the temperature only if this trajectory corresponds to the lifting of the device (1) near the head (16) of the patient (6).

2. The device according to claim 1, **characterized in that** the measurement window (11) of the housing (2) faces the direction opposite to the wrist (4).

3. The device according to claim 1 or 2, **characterized in that** the resolution of the accelerometer (10) falls in the range from 0.0005 g to 0.05 g.

4. The device according to claim 1 or 2 or 3, **characterized in that** the resolution of the passive infrared sensor (8) falls in the range from 0.01°C to 0.5°C.

5. The device according to any one of claims from 1 to 4, **characterized in that** the wireless communication module (9) contains a GSM/GPRS communication block and a WiFi communication block operating in the band band from 2,400 to 2,485 GHz.

6. The device according to one of claims from 1 to 5, **characterized in that** visual (12) and acoustic (13) signalling means are located on the housing (2).

7. The device according one of claims from 1 to 6, **characterized in that** microcontroller (7) control means (14) are located on the housing (2).

8. The device according to claim 6 or 7, **characterized in that** the visual signalling means (12) are RGB luminescent LEDs.

9. The device according to claim 6 or 7, **characterized in that** the acoustic signalling means (13) is a loudspeaker.

## Patentansprüche

1. Am Handgelenk zu tragende Vorrichtung (1) zur Messung der Körpertemperatur, die ein Gehäuse (2) mit Mitteln zur Befestigung (3) am Handgelenk (4) eines Patienten umfasst und im Inneren des Gehäuses (2) mindestens einen Mikrocontroller (7) und einen mit diesem signalverbundenen Temperaturwandler (8) und ein drahtloses Kommunikationsmodul (9) aufweist, wobei ein Passiv-Infrarot-Sensor der Temperaturwandler (8) ist, das Gehäuse (2) ein Messfenster (11) für den Passiv-Infrarot-Sensor (8) enthält, das Fenster (11) auf der Oberfläche des Gehäuses (2) angeordnet ist, die nicht für den Kontakt mit dem Handgelenk (4) bestimmt ist, und das Gehäuse (2) einen Beschleunigungsmesser (10) enthält, der mit dem Mikrocontroller (7) signalverbunden ist, **dadurch gekennzeichnet, dass** der Beschleunigungsmesser (10) so konfiguriert ist, dass er die Trajektorie der Vorrichtung (1) kontinuierlich aufzeichnet, und der Mikrocontroller (7) so konfiguriert ist, dass er die Temperatur nur dann abliest, wenn diese Trajektorie dem Anheben der Vorrichtung (1) in der Nähe des Kopfes (16) des Patienten (6) entspricht.

2. Vorrichtung nach Anspruch 1, **dadurch gekennzeichnet, dass** das Messfenster (11) des Gehäuses (2) in die dem Handgelenk (4) entgegengesetzte Richtung weist.

3. Vorrichtung nach Anspruch 1 oder 2, **dadurch gekennzeichnet, dass** die Auflösung des Beschleunigungsmessers (10) im Bereich von 0,0005 g bis 0,05 g liegt.

4. Vorrichtung nach Anspruch 1 oder 2 oder 3, **dadurch gekennzeichnet, dass** die Auflösung des Passiv-Infrarot-Sensors (8) im Bereich von 0,01 °C bis 0,5 °C liegt.

5. Vorrichtung nach einem der Ansprüche 1 bis 4, **dadurch gekennzeichnet, dass** das drahtlose Kommunikationsmodul (9) einen GSM/GPRS-Kommunikationsblock und einen WiFi-Kommunikationsblock enthält, die im Frequenzband von 2,400 bis 2,485 GHz arbeiten.

6. Vorrichtung nach einem der Ansprüche 1 bis 5, **dadurch gekennzeichnet, dass** am Gehäuse (2) optische (12) und akustische (13) Signalisierungsmittel angeordnet sind.

7. Vorrichtung nach einem der Ansprüche 1 bis 6, **dadurch gekennzeichnet, dass** sich am Gehäuse (2) Steuermittel (14) eines Mikrocontrollers (7) befinden.

8. Vorrichtung nach Anspruch 6 oder 7, **dadurch gekennzeichnet, dass** die optischen Signalisierungsmittel (12) RGB-Lumineszenz-LEDs sind.

9. Vorrichtung nach Anspruch 6 oder 7, **dadurch gekennzeichnet, dass** das akustische Signalisierungsmittel (13) ein Lautsprecher ist.

## Revendications

1. Un dispositif porté au poignet (1) pour mesurer la température corporelle, comprenant un boîtier (2) avec des moyens pour le fixer (3) au poignet (4) du patient, et contenant à l'intérieur du boîtier (2) au moins un microcontrôleur (7) et, relié par signal à celui-ci, un transmetteur de température (8) et un module de transmission sans fil (9), dans lequel un capteur infrarouge passif est le transmetteur de température (8), le boîtier (2) est muni d'une fenêtre de mesure (11) pour le capteur infrarouge passif (8), la fenêtre (11) est située sur la surface du boîtier (2) qui n'est pas destinée à entrer en contact avec le poignet (4) et le boîtier (2) est muni d'un accéléromètre (10) relié par signal au microcontrôleur (7), **caractérisé en ce que,** l'accéléromètre (10) est configuré pour enregistrer en continu la trajectoire du dispositif (1) et le microcontrôleur (7) est configuré pour lire la température uniquement si cette trajectoire correspond au soulèvement du dispositif (1) près de la tête (16) du patient (6).

2. Le dispositif suivant la revendication 1, **caractérisé en ce que** la fenêtre de mesure (11) du boîtier (2) est orientée dans la direction opposée au poignet (4).

3. Le dispositif suivant la revendication 1 ou 2, **caractérisé en ce que** la résolution de l'accéléromètre (10) est comprise entre 0,0005 g et 0,05 g.

4. Le dispositif suivant la revendication 1 ou 2 ou 3, **caractérisé en ce que** la résolution du capteur infrarouge passif (8) est comprise entre 0,01 °C et 0,5 °C.

5. Le dispositif suivant une des revendications de 1 à 4, **caractérisé en ce que** le module de transmission sans fil (9) contient un bloc de communication GSM/GPRS et un bloc de communication Wi-Fi fonctionnant sur une bande de 2,400 à 2,485 GHz.

6. Le dispositif suivant une des revendications de 1 à 5, **caractérisé en ce que** les moyens de signalisation visuelle (12) et sonore (13) sont situés sur le boîtier (2).

7. Le dispositif suivant une des revendications de 1 à 6, **caractérisé en ce que** les moyens de commande (14) du microcontrôleur (7) sont situés sur le boîtier (2).

8. Le dispositif suivant la revendication 6 ou 7, **caractérisé en ce que** les moyens de signalisation visuelle (12) sont des diodes électroluminescentes RVB.

9. Le dispositif suivant la revendication 6 ou 7, **caractérisé en ce que** le moyen de signalisation sonore (13) est un haut-parleur.
